# EUROPEAN PATENT SPECIFICATION

(11) **EP 0 946 541 B1**
(45) Date of publication and mention of the grant of the patent: **30.07.2003**
(21) Application number: 97911344.6
(22) Date of filing: 06.11.1997
(51) Int. Cl.: C07D 401/04, A61K 31/47, C07D 413/04, C07D 417/04, C07D 487/04, C07D 498/04, C07D 513/04, C07D 473/00, A61K 31/495

(54) **QUINOLINES AND THEIR THERAPEUTIC USE**
CHINOLINE UND DEREN THERAPEUTISCHE VERWENDUNG
QUINOLEINES ET LEUR UTILISATION THERAPEUTIQUE

(30) Priority: 06.11.1996 GB 9623163; 07.07.1997 GB 9714298
(43) Date of publication of application: 06.10.1999
(73) Proprietor: Darwin Discovery Limited, Slough, Berkshire SL1 3WE (GB)
(72) Inventor: DYKE, Hazel, Joan, Chiroscience Ltd., Cambridge CB4 4WE (GB); MONTANA, John, Gary, Chiroscience Ltd., Cambridge CB4 4WE (GB)
(74) Representative: Perry, Robert Edward
(86) International application number: GB9703047
(87) International publication number: WO98020007

(56) References cited:
- WO-A-94/12461
- ZARIF H. KHALIL ET AL.: "Synthesis of some oxazolo-, imidazolo-,selenadiazolo-,thiadiazolo-,aze tidinono- and thiazolidinono-8-hydroxyquinolines" JOURNAL INDIAN CHEMICAL SOCIETY, vol. 64, no. 1, 1987, CALCUTTA, pages 42-45, XP002040517

## Description

### Field of the Invention

The present invention relates to novel quinoline compounds and to their formulation and use as pharmaceuticals.

### Background of the Invention

EP-A-0498722 generically describes quinolines having amide and alkoxy substituents. US-A-5340811 discloses quinoline-5-sulfonamides as bronchodilators, for the treatment of asthma. US-A-4910193 discloses quinolines for the treatment of serotonin-induced gastrointestinal disturbances. US-A-4867301 generically discloses quinoline-5-sulfonamides as anti-allergy agents, e.g. for use in the treatment of asthma and chronic obstructive lung disease. JP-A-2/184673 also discloses quinoline-5-sulfonamides.

WO-A-9412461 discloses catechol diethers as selective phosphodiesterase (PDE) IV inhibitors. The modes of action of phosphodiesterases and also tumour necrosis factors (TNF), and the therapeutic utilities of inhibitors thereof, are described in WO-A-9720833 and PCT/GB97/01359-60, the contents of which are incorporated herein by reference. The two PCT Applications disclose quinoline derivatives.

### Summary of the invention

This invention provides novel compounds having therapeutic utility, in particular for the treatment of disease states associated with proteins which mediate cellular activity, for example by inhibiting TNF and/or PDE IV. According to the invention, the compounds are of formula (i): 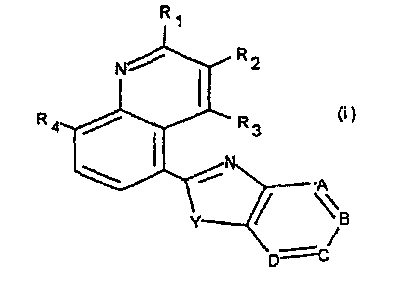 wherein A, B, C and D are the same or different and are each N, NO or CR₅, provided that not more than two are N or NO;
Y represents NR₇, O or S;
R₁, R₂ and R₃ are the same or different and each represents H, alkyl or CF₃
R₄ represents thioalkyl, or C₁₋₆ alkoxy or cycloalkoxy optionally substituted with one or more halogens;
R₅ represents H, halogen, alkyl, CF₃, hydroxy, OR₁₀, COR₁₁, SO₂R₁₁, SO₂NR₁₂R₁₃, NR₉R₁₀, CN, CO₂R₁₀, CONR₁₂R₁₃, NHSO₂CF₃ or tetrazolyl;
R₇ represents H or C₁₋₆ alkyl;
R₉ represents H, alkyl, aryl, heteroaryl, heterocyclo, arylalkyl, heteroarylalkyl, heterocycloalkyl, alkylcarbonyl, alkoxycarbonyl, arylcarbonyl, heteroarylcarbonyl, heterocyclocarbonyl, alkylsulphonyl, arylsulphonyl, heteroarylsulphonyl or heterocyclosulphonyl;
R₁₀, R₁₂ and R₁₃ are the same or different and each represents H or R₁₁, or NR₁₂R₁₃ represents a heterocyclic ring (such as morpholine or piperidine) optionally substituted with R₁₄;
R₁₁ represents alkyl, aryl, heteroaryl, heterocyclo, arylalkyl, heteroarylalkyl or heterocycloalkyl; and
R₁₄ represents aryl, arylalkyl or heteroarylalkyl;
and include pharmaceutically-acceptable salts thereof.

### Description of the Invention

Suitable pharmaceutically-acceptable salts are pharmaceutically-acceptable base salts and pharmaceutically-acceptable acid addition salts. Certain of the compounds of formula (i) which contain an acidic group form base salts. Suitable pharmaceutically-acceptable base salts include metal salts, such as alkali metal salts for example sodium salts, or organic amine salts such as that provided with ethylenediamine.

Certain of the compounds of formula (i) which contain an amino group form acid addition salts. Suitable acid addition salts include pharmaceutically-acceptable inorganic salts such as the sulphate, nitrate, phosphate, borate, hydrochloride and hydrobromide, and pharmaceutically-acceptable organic acid addition salts such as acetate, tartrate, maleate, citrate, succinate, benzoate, ascorbate, methanesulphate, α-ketoglutarate, α-glycerophosphate and glucose-1-phosphate. The pharmaceutically-acceptable salts of the compounds of formula (i) are prepared using conventional procedures.

It will be appreciated by those skilled in the art that some of the compounds of formula (i) may exist in more than one tautomeric form. This invention extends to all tautomeric forms.

It will also be appreciated that the compounds according to the invention can contain one or more asymmetrically substituted carbon atoms. The presence of one or more of these asymmetric centres in a compound of formula (i) can give rise to stereoisomers, and in each case the invention is to be understood to extend to all such stereoisomers, including enantiomers, and diastereoisomers and mixtures including racemic mixtures thereof.

When used herein, the term alkyl whether used alone or when used as a part of another group includes straight and branched chain alkyl groups containing up to 6 atoms. Cycloalkoxy means a cyclic alkyl group containing up to 6 carbon atoms and cycloalkoxy means a cycloalkyl-O- group. Alkoxy means an alkyl-O- group in which the alkyl group is as previously described. Aryl means an aromatic monocyclic or multicyclic carbocyclic group containing about 6 to 10 carbon atoms, Arylalkyl means an aryl-alkyl- group wherein the aryl and alkyl are as described herein. Heteroarylalkyl means a heteroaryl-alkyl group and heterocycloalkyl means a heterocyclo-alkyl group. Alkylcarbonyl means an alkyl-CO- group in which the alkyl group is as previously described. Arylcarbonyl means an aryl-CO- group in which the aryl group is as previously described. Heteroarylcarbonyl means a heteroaryl-CO- group and heterocyclocarbonyl means a heterocyclo-CO- group. Arylsulphonyl means an aryl-SO₂- group in which the aryl group is as previously described. Heteroarylsulphonyl means a heteroaryl-SO₂- group and heterocyclosulponyl means a heterocyclo-SO₂- group. Alkoxycarbonyl means an alkyloxy-CO- group in which the alkoxy group is as previously described. Alkylsulphonyl means an alkyl-SO₂- group in which the alkyl group is as previously described. Heteroaryl means about a 5 to about a 10 membered aromatic monocyclic or multicyclic hydrocarbon ring system in which one or more of the atoms in the ring system is an element other than carbon, chosen from amongst nitrogen, oxygen or sulphur. Heterocyclo means about a 5 to about a 10 membered saturated or partially saturated monocyclic or multicyclic hydrocarbon ring system in which one or more of the atoms in the ring system is an element other than carbon, chosen from amongst nitrogen, oxygen or sulphur. Halogen means fluorine, chlorine, bromine or iodine.

Compounds of the invention may be prepared by generally known processes from starting materials that are known or can readily be prepared by methods known to those of ordinary skill in the art. For example, procedures described in WO-A-9412461 and Biorganic & Med. Chem. Letters (1995) 5(17):1969, may be used, substituting for the catechol nucleus an appropriately substituted quinoline. The quinoline nucleus may be prepared as described in PCT/GB97/01359-60. Reference may also be made to the procedures given in J. Indian Chem. Soc. LXIV:42-45 (1987), and in the Examples below. Appropriate modifications will be evident to those of ordinary skill in the art.

It will be appreciated that functional groups such as amino, hydroxyl or carboxyl groups, and which it is desired to retain, may need to be in protected forms before any reaction is initiated. In such instances, removal of the protecting group may be the final step in a particular reaction. Suitable protecting groups for such functionality will be apparent to those skilled in the art. For specific details, see Protective Groups in Organic Synthesis, Wiley Interscience, TW Greene. Thus a process for preparing compounds of formula (i) containing an -OH group comprises deprotecting (for example by hydrogenolysis or hydrolysis) a compound of formula (i) which contains a corresponding -OP group wherein P represents a suitable protecting group (e.g. benzyl).

A compound of formula (i) may also be prepared by interconversion of other compounds of formula (i). For example, a compound in which R₃ contains an alcohol function may be prepared by reduction of a compound of formula (i) in which R₃ contains a carbonyl function. Compounds in which R₁-R₃ contain a CO-alkyl, CO-aryl, CO-heteroaryl, CO-alkylaryl, CO-alkylheteroaryl or CO-alkylheterocyclo group may be prepared from compounds in which R₁-R₃ contain a CN group by addition of a suitable organometallic agent (such as a Grignard reagent).

By way of further example, compounds in which R₁ -R₃ contain an oxime may be prepared from compounds in which R₁-R₃ contain a carbonyl group. This transformation may be carried out using any appropriate standard conditions known to those skilled in the art. 'Compounds of formula (i) in which R₁-R₃ contain a carbonyl group may be reduced using standard conditions known to those skilled in the art (for example with sodium borohydride in an appropriate solvent) to provide compounds in which R₁-R₃ contains an alcohol group. Compounds in which R₁-R₃ is alkyl may be prepared by reduction of compounds in which R₁-R₃ is CO-alkyl using standard conditions known to those skilled in the art (for example hydrazine hydrate in the presence of a suitable base in an appropriate solvent). Other transformations may be carried out on compounds of formula (i) in which R₁-R₃ contains a carbonyl group. Such transformations include, but are not limited to, reductive amination and alkylation. Any of the above transformations may be carried out either at the end of the synthesis or on an appropriate intermediate.

It will be appreciated that where a particular stereoisomer of formula (i) is required, this may be obtained by conventional resolution techniques such as high performance liquid chromatography or the synthetic processes herein described may by performed using the appropriate homochiral starting material.

The invention includes the prevention and treatment at TNF mediated disease or disease states, by which is meant any and all disease states in which TNF plays a role, either by production of TNF itself, or by TNF causing another cytokine to be released, such as but not limited to IL-1 or IL-6. A disease state in which IL-1, for instance, is a major component, and whose production or action is exacerbated or secreted in response to TNF, would therefore be considered a disease state mediated by TNF. As TNF-β (also known as lymphotoxin) has close structural homology with TNF-α (also known as cachectin), and since each induces similar biological responses and binds to the same cellular receptor, both TNF-α and TNF-β are inhibited by compounds of the present invention and thus are herein referred to collectively as "TNF" unless specifically delineated otherwise.

PDE IV inhibitors are useful in the treatment of a variety of allergic and inflammatory diseases, including: asthma, chronic bronchitis, atopic dermatitis, atopic eczema, urticaria, allergic rhinitis, allergic conjunctivitis, vernal conjunctivitis. inflammation of the eye, allergic responses in the eye, eosinophilic granuloma, psoriasis, Bechet's disease, erythematosis, anaphylactoid purpura nephritis, joint inflammation, arthritis, rheumatoid arthritis and other arthritic conditions such as rheumatoid spondylitis and osteoarthritis, septic shock, ulcerative colitis, Crohn's disease, reperfusion injury of the myocardium and brain, chronic glomerulonephritis, endotoxic shock and adult respiratory distress syndrome. In addition, PDE IV inhibitors are useful in the treatment of diabetes insipidus and conditions associated with cerebral metabolic inhibition, such as cerebral senility, senile dementia (Alzheimer's disease), memory impairment associated with Parkinson's disease, depression and multi-infarct dementia. PDE IV inhibitors are also useful in conditions ameliorated by neuroprotectant activity, such as cardiac arrest, stroke and intermittent claudication. Additionally, PDE IV inhibitors could have utility as gastroprotectants. A special embodiment of the therapeutic methods of the present invention is the treatment of asthma.

The viruses contemplated for treatment herein are those that produce TNF as a result of infection, or those which are sensitive to inhibition, such as by decreased replication, directly or indirectly, by the TNF inhibitors of Formula (i). Such viruses include, but are not limited to HIV-1, HIV-2 and HIV-3, cytomegalovirus (CMV), influenza, adenovirus and the Herpes group of viruses, such as, but not limited to, *Herpes zoster* and *Herpes simplex.*

This invention more specifically relates to a method of treating a mammal, afflicted with a human immunodeficiency virus (HIV), which comprises administering to such mammal an effective TNF-inhibiting amount of a compound of Formula (i) or a pharmaceutically-acceptable salt thereof.

The compounds of this invention may also be used in association with the veterinary treatment of animals, other than humans, in need of inhibition of TNF production. TNF-mediated diseases for treatment, therapeutically or prophylactically, in animals include disease states such as those noted above, but in particular viral infections. Examples of such viruses include, but are not limited to feline immunodeficiency virus (FIV) or other retroviral infection such as equine infectious anaemia virus, caprine arthritis virus, visna virus, maedi virus and other lentiviruses.

The compounds of this invention are also useful in treating parasite, yeast and fungal infections, where such yeast and fungi are sensitive to upregulation by TNF or will elicit TNF production *in vivo*. A preferred disease state for treatment is fungal meningitis.

The compounds of formula (i) are preferably in pharmaceutically-acceptable form. By pharmaceutically-acceptable form is meant, *inter alia*, a pharmaceutically-acceptable level of purity excluding normal pharmaceutical additives such as diluents and carriers, and including no material considered toxic at normal dosage levels. A pharmaceutically-acceptable level of purity will generally be at least 50% excluding normal pharmaceutical additives, preferably 75%, more preferably 90% and still more preferably 95%. When used herein the term "pharmaceutically-acceptable" encompasses materials suitable for both human and veterinary use.

A compound of formula (i) or where appropriate a pharmaceutically-acceptable salt thereof and/or a pharmaceutically-acceptable solvate thereof, may be administered *per se* or, preferably, as a pharmaceutical composition also comprising a pharmaceutically-acceptable carrier.

Accordingly, the present invention provides a pharmaceutical composition comprising a compound of formula (i) or where appropriate a pharmaceutically-acceptable salt thereof and/or a pharmaceutically-acceptable solvate thereof, and a pharmaceutically-acceptable carrier.

The active compound may be formulated for administration by any suitable route, the preferred route depending upon the disorder for which treatment is required, and is preferably in unit dosage form or in a form that a human patient may administer to himself in a single dosage. Advantageously, the composition is suitable for oral, rectal, topical, parenteral administration or through the respiratory tract. Preparations may be designed to give slow release of the active ingredient.

The term parenteral as used herein includes subcutaneous injections, intravenous, intramuscular, intrasternal injection or infusion techniques. In addition to the treatment of warm-blooded animals such as mice, rats, horses, cattle, sheep, dogs, cats, etc, the compounds of the invention are effective in the treatment of humans.

The compositions of the invention may be in the form of tablets, capsules, sachets, vials, powders, granules, lozenges, suppositories, reconstitutable powders, or liquid preparations such as oral or sterile parenteral solutions or suspensions. Topical formulations are also envisaged where appropriate.

In order to obtain consistency of administration it is preferred that a composition of the invention is in the form of a unit dose. Unit dose presentation forms for oral administration may be tablets and capsules and may contain conventional excipients such as binding agents, for example syrup, acacia, gelatin, sorbitol, tragacanth, or polyvinylpyrrolidone; fillers for example microcrystalline cellulose, lactose, sugar, maize-starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically-acceptable wetting agents such as sodium lauryl sulphate.

Solid oral compositions may be prepared by conventional methods of blending, filling, tabletting or the like. Repeated blending operations may be used to distribute the active agent throughout those compositions employing large quantities of fillers.

Such operations are of course conventional in the art. The tablets may be coated according to methods well known in normal pharmaceutical practice, in particular with an enteric coating.

Oral liquid preparations may be in the form of, for example, emulsions, syrups or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia, nonaqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

Compositions may also suitably be presented for administration to the respiratory tract as a snuff or an aerosol or solution for a nebuliser, or as a microfine powder for insufflation, alone or in combination with an inert carrier such as lactose. In such a case the particles of active compound suitably have diameters of less than 50 µm, such as from 0.1 to 50 µm, preferably less than 10 µm, for example from 1 to 10 µm, 1 to 5 µm or from 2 to 5 µm. Where appropriate, small amounts of other anti-asthmatics and bronchodilators for example sympathomimetic amines such as isoprenaline, isoetharine, salbutamol, phenylephrine and ephedrine; corticosteroids such as prednisolone and adrenal stimulants such as ACTH may be included.

For parenteral administration, fluid unit dosage forms are prepared utilizing the compound and a sterile vehicle, and, depending on the concentration used, can be either suspended or dissolved in the vehicle. In preparing solutions, the compound can be dissolved in water for injection and filter-sterilised before filling into a suitable vial or ampoule and sealing.

Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

Compounds of formula (i), or if appropriate a pharmaceutically-acceptable salt thereof and/or a pharmaceutically-acceptable solvate thereof, may also be administered as a topical formulation in combination with conventional topical excipients.

Topical formulations may be presented as, for instance, ointments, creams or lotions, impregnated dressings, gels, gel sticks, spray and aerosols, and may contain appropriate conventional additives such as preservatives, solvents to assist drug penetration and emollients in ointments and creams. The formulations may contain compatible conventional carriers, such as cream or ointment bases and ethanol or oleyl alcohol for lotions.

Suitable cream, lotion, gel, stick, ointment, spray or aerosol formulations that may be used for compounds of formula (i) or if appropriate a pharmaceutically-acceptable salt thereof, are conventional formulations well known in the art, for example, as described in standard text books such as Harry's Cosmeticology published by Leonard Hill Books, Remington's Pharmaceutical Sciences, and the British and US Pharmacopoeias.

Suitably, the compound of formula (i), or if appropriate a pharmaceutically-acceptable salt thereof, will compromise from about 0.5 to 20% by weight of the formulation, favourably from about 1 to 10%, for example 2 to 5%.

The dose of the compound used in the treatment of the invention will vary in the usual way with the seriousness of the disorders, the weight of the sufferer, and the relative efficacy of the compound. However, as a general guide suitable unit doses may be 0.1 to 1000 mg, such as 0.5 to 200, 0.5 to 100 or 0.5 to 10 mg, for example 0.5, 1, 2, 3, 4 or 5 mg; and such unit doses may be administered more than once a day, for example 2, 3, 4, 5 or 6 times a day, but preferably 1 or 2 times per day, so that the total daily dosage for a 70 kg adult is in the range of about 0.1 to 1000 mg, that is in the range of about 0.001 to 20 mg/kg/day, such as 0.007 to 3, 0.007 to 1.4, 0.007 to 0.14 or 0.01 to 0.5 mg/kg/day, for example 0.01, 0.02, 0.04, 0.05, 0.06, 0.08, 0.1 or 0.2 mg/kg/day, and such therapy may extend for a number of weeks or months.

The following Examples illustrate the invention.

### Intermediate 1 8-Methoxyquinoline-5-carboxylic acid chloride, hydrochloride

A suspension of 8-methoxyquinoline-5-carboxylic acid (5.0 g) in dichloromethane (50 ml) was cooled to 0°C. Oxalyl chloride (4.29 ml) was added cautiously and then dry dimethylformamide (8 drops) was added dropwise. The mixture was stirred at 0°C for 1h and then at room temperature for 24h. The solvent was removed *in vacuo* and the residue azeotroped with a small amount of toluene. The title compound was obtained as a brown solid (5.18 g).
NMR (200MHz, d₆-DMSO) δ 9.96(d, 1H), 9.18(dd, 1H), 8.52(d, 1H), 8.12(dd, 1H), 7.61(d, 1H), 4.20(s, 3H).

### Intermediate 2 8-Methoxyquinoline-5-[N-(2-amino-5-carbomethoxyphenyl)]-carboxamide

A solution of methyl 3,4-diaminobenzoate (1.27 g) in dry pyridine (10 ml) was cooled to 0°C under nitrogen. A suspension of 8-methoxyquinoline-5-carbonyl chloride, hydrochloride (1.97 g) in dry tetrahydrofuran was added, the resultant suspension was allowed to warm to room temperature and then stirred for 16h. The resultant suspension was heated at 60°C for 16h and then cooled to room temperature. The mixture was concentrated *in vacuo* and ethyl acetate (100 ml) and 2M hydrochloric acid (50 ml) were added. The resultant precipitate was collected by filtration to provide the title compound as a brown solid (919 mg).
TLC R_{f} 0.52 (10% methanol in dichloromethane).

### Intermediate 3 8-Methoxy-2-trifluoromethylquinoline-5-[N-(2-amino-5-carbomethoxyphenyl)]carboxamide

A solution of methyl 3,4-diaminobenzoate (1.0 g) in dry pyridine (25 ml) and triethylamine (1.7 ml) was cooled to 0°C under nitrogen. A suspension of 8-methoxy-2-trifluoromethylquinoline-5-carbonyl choride, hydrochloride (1.8 g; Intermediate 35 of PCT/GB97/01359) in dry tetrahydrofuran (40 ml) was added; the resultant suspension was allowed to warm to room temperature and then stirred overnight. The solvent was removed *in vacuo* and the residue partitioned between ethyl acetate (200 ml) and water (200 ml). The solid precipitate was removed by filtration and dried *in vacuo* at 45°C to yield the title compound as an off white solid (1.34 g).
Mass spectrum (EI) 420 [M+H]⁺

### Intermediate 4 8-Methoxy-2-methylquinoline-5-[N-(2-hydroxy-5-methoxyphenyl)]carboxamide

A solution of 2-amino-4-methoxyphenol (3.8 g) in dry dimethylformamide (200 ml) was stirred at room temperature under nitrogen. Sodium hydride (3.9 g) was added and stirring continued for 2h. 8-Methoxy-2-methylquinoline-5-carbonyl choride, hydrochloride (6.2 g; Intermediate 28 of PCT/GB97/01359) was added, and the resultant mixture was stirred overnight. The solvent was removed *in vacuo* and the residue purified by flash chromatography eluting with ethyl acetate to give the title compound as a brown solid (0.86 g).
TLC R_{f} 0.30 (ethyl acetate)

### Intermediate 5 2-(8-Methoxy-2-trifluoromethylquinolin-5-yl)-1H-benzimidazole-5-carboxylic acid, methyl ester

8-Methoxy-2-trifluoromethylquinoline-5-[N-(2-amino-5-carbomethoxyphenyl)]carboxamide (1.3 g) was suspended in phosphorus oxychloride (10 ml) and heated at reflux for 4h. The mixture was cooled to room temperature and concentrated *in vacuo*. The residue was neutralised by the addition of 46% aqueous sodium hydroxide solution and the resultant solid was collected by filtration and dried *in vacuo*. The title compound was obtained as a grey solid (1.3 g).
TLC R_{f} 0.55 (ethyl acetate)

### Example 1 2-(8-Methoxyquinolin-5-yl)-1H-benzimidazole-5-carboxylic acid, methyl ester

8-Methoxyquinoline-5-[*N*-(2-amino-5-carbomethoxyphenyl)]carboxamide (919 mg) was suspended in phosphorus oxychloride (10 ml) and heated at reflux for 4h. The mixture was cooled to room temperature and concentrated *in vacuo*. The residue was basified to pH 8 by the addition of saturated aqueous sodium hydrogen carbonate solution and the resultant solid was collected by filtration and dried *in vacuo*. The title compound was obtained as a light brown solid (850 mg).
NMR (400MHz, d₆-DMSO) δ 9.79(d, 1H), 8.94(d, 1H), 8.38(d, 1H), 8.25(d, 1H), 7.88(d, 1H), 7.73(d, 1H), 7.70(d, 1H), 7.40(d, 1H), 4.08(s, 3H), 3.89(s, 3H).

### Example 2 2-(8-Methoxyquinolin-5-yl)-1H-benzimidazole-5-carboxylic acid, methyl ester dihydrochloride

A sample of 2-(8-methoxyquinolin-5-yl)-1H-benzimidazole-5-carboxylic acid, methyl ester (45 mg) was dissolved in 2M hydrochloric acid (3 ml), the resultant solution filtered and the filtrate concentrated *in vacuo*, The residue was triturated with dichloromethane and dried *in vacuo* to provide the title compound as a yellow solid (34 mg).
NMR (200MHz, d₆-DMSO) δ 9.82(d, 1H), 9.10(d, 1H), 8.40(d, 1H), 8.32(bs, 1H), 7.90(bd, 2H), 7.87(d, 1H), 7.66(d, 1H), 4.18(s, 3H), 3.91(s, 3H).

### Example 3 2-(8-Methoxyquinolin-5-yl)-1H-benzimidazole-5-carboxylic acid dihydrochloride

A mixture of 2-(8-methoxyquinolin-5-yl)-1H-benzimidazole-5-carboxylic acid, methyl ester (730 mg), 1M sodium hydroxide solution (40 ml) and ethanol (40 ml) was heated at reflux for 2h and then stirred at room temperature for 16h. The mixture was concentrated *in vacuo* and the residue acidified to pH 1 with concentrated hydrochloric acid and diluted with water (20 ml). The resultant solid was collected by filtration and then dissolved in methanol (30 ml). The solution was concentrated in *vacuo* and the resultant yellow solid dried over silica to provide the title compound (600 mg).
TLC R_{f} 0.15 (1% acetic acid, 5% methanol in ethyl acetate)
Mass spectrum (CI) 320 [M+H]⁺

### Example 4 2-(8-Methoxy-2-methylquinolin-5-yl)-5-methoxy-1H-benzimidazole

A solution of4-methoxy-1,2-phenylenediamine (3.0 g) in dry pyridine (50 ml) and triethylamine (5.5 ml) was cooled to 0°C under nitrogen. A suspension of 8-methoxy-2-methylquinoline-5-carbonyl choride, hydrochloride (5.4 g; Intermediate 28 of PCT/GB97/01359) in dry tetrahydrofuran (60 ml) was added; the resultant suspension was allowed to warm to room temperature and then stirred overnight. The solvent was removed *in vacuo* and the residue partitioned between ethyl acetate (2x200 ml) and water (300 ml). The solid precipitate was removed by filtration and dried *in vacuo* at 50°C to yield an off white solid (4.25 g). The crude amide (1.0 g) was suspended in phosphorus oxychloride (10 ml) and heated at reflux for 4h. The mixture was cooled to room temperature and concentrated *in vacuo*. The residue was diluted with water and left to stand for 2 days. The solution was basified to pH14 by the addition of 46% aqueous sodium hydroxide solution and the resultant brown solid was collected by filtration and dried *in vacuo* to give a brown solid. Purification by flash chromatography eluting with 10% methanol/1% triethylamine/ethyl acetate followed by trituration with hexane gave the title compound as a beige solid (0.12 g).
TLC R_{f} 0.32 (10% methanol in ethyl acetate)
m.p. 145.5-147.5°C

The following compounds were prepared in a similar manner using the appropriate starting materials.

### Example 5 2-(8-Methoxy-2-methylquinolin-5-yl)-5-aza-1H-benzimidazole

Prepared from 3,4-diaminopyridine; the title compound was obtained as a light brown solid (0.37 g).
TLC R_{f} 0.54 (12% methanol in dichloromethane)
m.p. 150°C (dec.)

### Example 6 2-(8-Methoxy-2-methylquinolin-5-yl)-1H-benzimidazole-5-carboxylic acid, methyl ester

Prepared from methyl 3,4-diaminobenzoate; purification by flash chromatography eluting with ethyl acetate gave the title compound as a pale orange solid (0.17 g).
TLC R_{f} 0.25 (ethyl acetate)
m.p. 165-167°C

### Example 7 2-(8-Methoxy-2-trifluoromethylquinolin-5-yl)-5-methoxy-1H-benzimidazole

Prepared from 8-methoxy-2-trifluoromethylquinoline-5-carbonyl choride, hydrochloride (Intermediate 35 of PCT/GB97/01359) and 4-methoxy-1,2-phenylenediamine; purification by column chromatography eluting with 50%-70% ethyl acetate in hexane afforded the title compound as a pale yellow solid (1.32 g).
TLC R_{f} 0.14 (50% ethyl acetate in hexane)
m.p. 131-133°C.

### Example 8 2-(8-Methoxy-2-methylquinolin-5-yl)-5-methoxybenzoxazole

8-Methoxy-2-methylquinoline-5-[*N*-(2-hydroxy-5-methoxyphenyl)]carboxamide (0.86 g) was suspended in phosphorus oxychloride (8.8 ml) and heated at reflux for 5h. The mixture was cooled to room temperature and concentrated *in vacuo*. The residue was diluted with water and neutralised by the addition of IN aqueous sodium hydroxide solution. The aqueous phase was extracted with dichloromethane (5x50 ml), dried (magnesium sulphate) and the solvent removed *in vacuo* to give a brown glass. Purification by flash chromatography eluting with ethyl acetate followed by trituration with acetone gave the title compound as a white solid (1.3 mg).
TLC R_{f} 0.37 (ethyl acetate)
NMR (200MHz, CDCl₃) δ 2.9 (s, 3H), 3.9 (s, 3H), 4.2 (s, 3H), 7.0 (dd, 1H), 7.2 (d, 1H), 7.4 (d, 1H), 7.6 (dd, 2H), 8.4 (d, 1H), 9.9 (d, 1H).

### Example 9 2-(8-Methoxy-2-trifluoromethylquinolin-5-yl)-5-methoxy-1-methyl-benzimidazole and 2-(8-Methoxy-2-trifluoromethylquinolin-5-yl)-6-methoxy-1-methyl-benzimidazole

Sodium hydride (32 mg) was added to 2-[8-methoxy-2-trifluoromethylquinolin-5-yl)]-5-methoxy-1H-benzimidazole (0.25 g) in *N,N*-dimethylformamide (5 ml) at room temperature under an inert atmosphere. After stirring for 25 minutes iodomethane (0.06 ml) was added and the reaction stirred at room temperature overnight. The solvent was removed *in vacuo* and the residue partitioned between dichloromethane (2x45 ml) and water (45 ml). The combined organic phases were dried over magnesium sulphate and preadsorbed onto silica *in vacuo*. Purification by column chromatography eluting with 75% ethyl acetate in hexane afforded the title compound as a pale yellow solid (0.23 g) as a 1:1 mixture of the two isomers. TLC R_{f} 0.49 (75% ethyl acetate in hexane) Mass spectrum (CI) 388 [M+H]⁺

### Example 10 2-(8-Methoxy-2-trifluoromethylquinolin-5-yl)-1H-benzimidazole-5-carboxylic acid

Sodium hydroxide (1M, 40 ml) was added to a stirred suspension of 2-(2-trifluoromethyl-8-methoxyquinolin-5-yl)-1H-benzimidazole-5-carboxylic acid, methyl ester (1 g) in ethanol (40 ml) at room temperature. The reaction was heated to 80°C for 4h and cooled to room temperature. The ethanol was removed *in vacuo* and the remaining aqueous phase acidified to pH 5 with glacial acetic acid. The resulting solid was collected by filtration and dried *in vacuo* to give the titled compound as a brown solid (0.88 g).
m.p. 249°C (dec.)
Mass spectrum (CI) 388 [M+H]⁺

The following compound was prepared in a similar manner using the appropriate starting materials.

### Example 11 2-(8-Methoxy-2-methylquinolin-5-yl)-1H-benzimidnzole-5-carboxylic acid

Prepared from 2-(8-Methoxy-2-methylquinolin-5-yl)-1H-benzimidazole-5-carboxylic acid, methyl ester; the title compound was obtained as a white solid (35 mg).
TLC R_{f} 0.16 (10% methanol in dichloromethane)
m.p. 205-207°C (dec.)

### Example 12 2-(8-Methoxy-2-trifluoromethylquinolin-5-yl)-1H-benzimidazole-5-carboxamide

Ammonia (0.5M in 1,4-dioxane, 3.9 ml) was added to a stirred solution of 2-(8-methoxy-2-trifluoromethylquinolin-5-yl)-1H-benzimidazole-5-carboxylic acid (0.15 g) in 1,4-dioxane (30 ml) at room temperature under an inert atmosphere, 1-(3-Dimethylaminopropyl)-3-ethylcarbodiimide hydrochloride (0.11 g) was added followed by 4-dimethylaminopyridine (36 mg) and the reaction stirred at room temperature overnight. The solvent was removed *in vacuo* and the residue partitioned between ethyl acetate (2x40 ml) and water (50 ml). The combined organic phases were dried over magnesium sulphate and preadsorbed onto silica in *vacuo.* Purification by column chromatography eluting with 10% methanol in ethyl acetate afforded the title compound as a cream solid (20 mg).
TLC R_{f} 0.62 (10% methanol in ethyl acetate)
m.p. 205-207°C

### Assay Methods

The assays used to confirm the phosphodiesterase IV inhibitory activity of compounds of formula (I) are standard assay procedures as disclosed by Schilling *et al*, Anal. Biochem. 216:154 (1994), Thompson and Strada, Adv. Cycl. Nucl. Res. 8:119 (1979) and Gristwood and Owen, Br. J. Pharmacol. 87:91P (1986).

Compounds of formula (i) have exhibited activity at levels consistent with those believed to be useful in treating phosphodiesterase IV related disease states in those assays.

The ability of compounds of formula (i) to inhibit TNF production in human peripheral blood mononuclear cells (PMBC's) is measured as follows. PMBC's are prepared from freshly taken blood or "Buffy coats" by standard procedures. Cells are plated out in RPMI1640 +1% foetal calf serum in the presence and absence of inhibitors. LPS (100 ng/ ml) is added and cultures are incubated for 22 h at 37°C in an atmosphere of 95% air/5% CO₂. Supernatants are tested for TNFα by ELISA using commercially available kits.

*In vivo* activity in a skin eosinophilia model is determined by using the methods described by Hellewell *et al*, Br. J. Pharmacol. 111:811 (1994) and Br. J. Pharmacol. 110:416 (1993). Activity in a lung model is measured using the procedures described by Kallos and Kallos, Int. Archs. Allergy Appl. Immunol. 73:77 (1984), and Sanjar *et al*, Br. J. Pharmacol. 99:679 (1990).

An additional lung model, which allows measurement of inhibition of the early and late-phase asthmatic responses and also the inhibition of airway hyperreactivity, is described by Broadley *et al*, Pulmonary Pharmacol. 7:311 (1994), J. Immunological Methods 190:51 (1996) and British J. Pharmacol. 116:2351 (1995). Compounds of the invention show activity in this model.

### Abbreviations

- LPS: Lipopolysaccharide (endotoxin)
- ELISA: Enzyme linked immunosorbent assay

## Claims

1. A compound of formula (i) 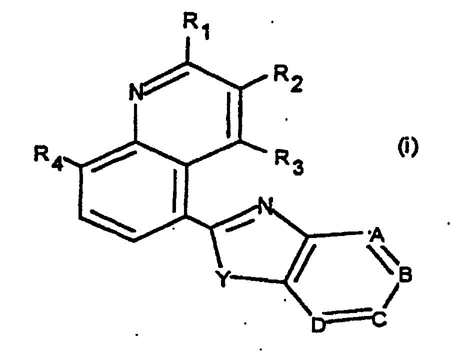 wherein A, B, C and D are the same or different and are each N, NO or CR₅, provided that not more than two are N or NO;
Y represents NR₇, O or S;
R₁, R₂ and R₃ are the same or different and each represents H, alkyl or CF₃;
R₄ represents thioalkyl, or C₁₋₆ alkoxy or cycloalkoxy optionally substituted with one or more halogens;
R₅ represents H, halogen, alkyl, CF₃, hydroxy, OR₁₀, COR₁₁, SO₂R₁₁, SO₂NR₁₂R₁₃, NR₉R₁₀, CN, CO₂R₁₀, CONR₁₂R₁₃, NHSO₂CF₃ or tetrazolyl;
R₇ represents H or C₁₋₆ alkyl;
R₉ represents H, alkyl, aryl, heteroaryl, heterocyclo, arylalkyl, heteroarylalkyl, heterocycloalkyl, alkylcarbonyl, alkoxycarbonyl, arylcarbonyl, heteroarylcarbonyl, heterocyclocarbonyl, alkylsulphonyl, arylsulphonyl, heteroarylsulphonyl or heterocyclosulphonyl;
R₁₀, R₁₂ and R₁₃ are the same or different and each represents H or R₁₁, or NR₁₂R₁₃ represents a heterocyclic ring optionally substituted with R₁₄;
R₁₁ represents alkyl, aryl, heteroaryl, heterocyclo, arylalkyl, heteroarytalkyl or heterocycloalkyl; and
R₁₄ represents aryl, arylalkyl or heteroarylalkyl;
or a pharmaceutically-acceptable salt thereof.

2. A compound of claim 1, wherein R₄ is thioalkyl or optionally-substituted alkoxy; and A, B, C and D are each N or CR₅, provided that not more than one is N.

3. A compound of claim 1 or claim 2, wherein R₄ is optionally-substituted alkoxy.

4. A. compound of claim 3,
wherein one of A, B, C and D is CR₅ and the others are CH;
R₁, R₂ and R₃ are the same or different and are each H or alkyl;
R₅ is COOR₁₀, CONR₁₂, NHSO₂CF₃ or tetrazolyl; and
R₁₂ and R₁₃ are each H or R₁₁.

5. A compound of claim 1, which is 2-(8-methoxyquinolin-5-yl)-1H-benzimidazole-5-carboxylic acid as its methyl ester, dihydrochloride or methyl ester dihydrochloride.

6. A compound of claim 1, which is
2-(8-methoxy-2-methylquinolin-5-yl)-5-methoxy-1H-benzimidazole,
2-(8-methoxy-2-trifluoromethylquinolin-5-yl)-5-methoxy-1H-benzimidazole,
2-(8-methoxy-2-trifluoromethylquinolin-5-yl)-5-methoxy-1-methyl-benzimidazole,
or
2-(8-methoxy-2-trifluoromethyl-quinolin-5-yl)-6-methoxy-1-methylbenzimidazole.

7. A compound of claim 1, which is
2-(8-methoxy-2-methylquinolin-5-yl)-5-aza-1H-benzimidazole,
2-(8-methoxy-2-methylquinolin-5-yl)-1H-benzimidazole-5-carboxylic acid, methyl ester,
2-(8-methoxy-2-methylquinolin-5-yl)-5-methoxybenzoxazole,
2-(8-methoxy-2-trifluoromethylquinolin-5-yl)-1H-benzimidazole-5-carboxylic acid,
2-(8-methoxy-2-methylquinolin-5-yl)-1H-benzimidazole-5-carboxylic acid, or
2-(8-methoxy-2-trifluoromethylquinolin-5-yl)-1H-benzimidazole-5-carboxamide.

8. A compound of any preceding claim, in the form of an enantiomer thereof.

9. A pharmaceutical composition for therapeutic use comprising a compound of any preceding claim and a pharmaceutically-acceptable carrier or excipient.

10. Use of a compound of any of claims 1 to 8, for the manufacture of a medicament for use in the treatment of a disease state that is capable of being modulated by inhibition of phosphodiesterase IV or Tumour Necrosis Factor, or, that is a pathological condition associated with a function of phosphodiesterase IV, eosinophil accumulation or a function of the eosinophil.

11. The use of claim 10, wherein the disease state is an inflammatory disease or autoimmune disease.

12. The use of claim 10, wherein the disease state is selected from asthma, chronic bronchitis, chronic pulmonary inflammatory disease, chronic obstructive airways disease, atopic dermatitis, allergic rhinitis, psoriasis, arthritis, rheumatoid arthritis, joint inflammation, ulcerative colitis, Crohn's disease, atopic eczema, stroke, bone resorption disease, multiple sclerosis and inflammatory bowel disease.

13. The use of claim 10, wherein the disease state is selected from urticaria, allergic conjunctivitis, vernal conjunctivitis, inflammation of the eye, allergic responses in the eye, eosinophilic granuloma, gouty arthritis and other arthritic conditions, adult respiratory distress syndrome, diabetes insipidus, keratosis, cerebral senility, multi-infarct dementia, senile dementia, memory impairment associated with Parkinson's disease, depression, cardiac arrest, intermittent claudication, rheumatoid spondylitis, osteoarthritis, sepsis, septic shock, endotoxic shock, gram negative sepsis, toxic shock syndrome, acute respiratory distress syndrome, cerebral malaria, silicosis, pulmonary sarcoidosis, reperfusion injury, graft vs host reaction, allograft rejection, infection-related fever or myalgia, malaria, HIV, AIDS, ARC, cachexia, keloid formation, scar tissue formation, pyresis, systemic lupus erythematosus, type 1 diabetes mellitus, Bechet's disease, anaphylactoid purpura nephritis, chronic glomerulonephritis, leukaemia, tarditive dyskinesia, yeast or fungal infection, conditions requiring gastroprotection, and neurogenic inflammatory disease associated with irritation and pain.

14. The use of claim 11, wherein the disease state is asthma.

15. The use of claim 11, wherein the disease state is chronic obstructive airways disease or chronic bronchitis.

## Patentansprüche

1. Verbindung der Formel (i) 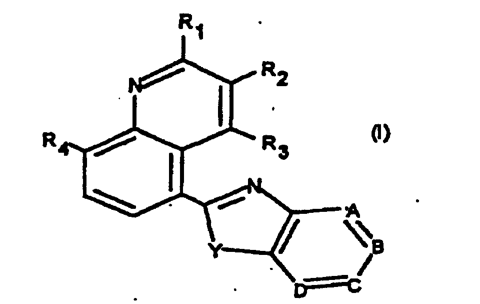 worin A, B, C und D gleich oder verschieden sind und jeweils N, NO oder CR₅ sind, mit der Maßgabe, dass nicht mehr als zwei N oder NO sind;
Y NR₇, O oder S ist;
R₁, R₂ und R₃ gleich oder verschieden sind und jeweils-H, Alkyl oder CF₃ darstellen;
R₄ Thioalkyl oder C₁₋₆-Alkoxy oder Cycloalkoxy, gegebenenfalls substituiert mit einem oder mehreren Halogenen, darstellt;
R₅ H, Halogen, Alkyl, CF₃, Hydroxy, OR₁₀, COR₁₁, SO₂R₁₁, SO₂NR₁₂R₁₃, NR₉R₁₀, CN, CO₂R₁₀, CONR₁₂R₁₃, NHSO₂CF₃ oder Tetrazolyl ist;
R₇ H oder C₁₋₆-Alkyl ist;
R₉ H, Alkyl, Aryl, Heteroaryl, Heterocyclo, Arylalkyl, Heteroarylalkyl, Heterocycloalkyl, Alkylcarbonyl, Alkoxycarbonyl, Arylcarbonyl, Heteroarylcarbonyl, Heterocyclocarbonyl, Alkylsulfonyl, Arylsulfonyl, Heteroarylsulfonyl oder Heterocyclosulfonyl ist;
R₁₀, R₁₂ und R₁₃ gleich oder verschieden sind und jeweils H oder R₁₁ sind oder NR₁₂R₁₃ einen heterocyclischen Ring, gegebenenfalls substituiert mit R₁₄, darstellt;
R₁₁ Alkyl, Aryl, Heteroaryl, Heterocyclo, Arylalkyl, Heteroarylalkyl oder Heterocycloalkyl darstellt; und
R₁₄ Aryl, Arylalkyl oder Heteroarylalkyl ist;
oder ein pharmazeutisch verträgliches Salz davon.

2. Verbindung nach Anspruch 1, worin R₄ Thioalkyl oder gegebenenfalls substituiertes Alkoxy ist und A, B, C und D jeweils N oder CR₅ sind, mit der Maßgabe, dass nicht mehr als eines N ist.

3. Verbindung nach Anspruch 1 oder Anspruch 2, worin R₄ gegebenenfalls substituiertes Alkoxy ist.

4. Verbindung nach Anspruch 3, worin eines von A, B, C und D CR₅ ist und die anderen CH sind, R₁, R₂ und R₃ gleich oder verschieden sind und jeweils H oder Alkyl sind, R₅ COOR₁₀, CONR₁₂, NHSO₂CF₃ oder Tetrazolyl ist und R₁₂ und R₁₃ jeweils H oder R₁₁ sind.

5. Verbindung nach Anspruch 1, welche 2-(8-Methoxychinolin-5-yl)-1H-benzimidazol-5-carbonsäure als deren Methylester, Dihydrochlorid oder Methylesterdihydrochlorid ist.

6. Verbindung nach Anspruch 1, welche
2-(8-Methoxy-2-methylchinolin-5-yl)-5-methoxy-1H-benzimidazol,
2-(8-Methoxy-2-trifluormethylchinolin-5-yl)-5-methoxy-1 H-benzimidazol,
2-(8-Methoxy-2-trifluormethylchinolin-5-yl)-5-methoxy-1-methylbenzimidazol oder
2-(8-Methoxy-2-trifluormethylchinolin-5-yl)-6-methoxy-1-methylbenzimidazol ist.

7. Verbindung nach Anspruch 1, welche
2-(8-Methoxy-2-methylchinolin-5-yl)-5-aza-1 H-benzimidazol,
2-(8-Methoxy-2-methylchinolin-5-yl)-1H-benzimidazol-5-carbonsäuremethylester,
2-(8-Methoxy-2-methylchinolin-5-yl)-5-methoxybenzoxazol,
2-(8-Methoxy-2-trifluormethylchinolin-5-yl)-1H-benzimidazol-5-carbonsäure,
2-(8-Methoxy-2-methylchinolin-5-yl)-1H-benzimidazol-5-carbonsäure oder
2-(8-Methoxy-2-trifluormethylchinolin-5-yl)-1H-benzimidazol-5-carboxamid ist.

8. Verbindung nach irgendeinem vorhergehenden Anspruch in Form eines Enantiomers davon.

9. Pharmazeutische Zusammensetzung für die therapeutische Verwendung umfassend eine Verbindung nach irgendeinem vorhergehenden Anspruch und einen pharmazeutisch verträglichen Träger oder Exzipienten.

10. Verwendung einer Verbindung nach irgendeinem der Ansprüche 1 bis 8 zur Herstellung eines Arzneimittels zur Verwendung bei der Behandlung eines Krankheitszustands, der durch Inhibierung von Phosphodiesterase IV oder Tumor-Nekrose-Faktor moduliert werden kann, oder der ein pathologischer Zustand ist, welcher mit einer Funktion von Phosphodiesterase IV, Eosinophil-Akkumulation oder einer Funktion des Eosinophils assoziiert ist.

11. Verwendung nach Anspruch 10, worin der Krankheitszustand eine Entzündungskrankheit oder eine Autoimmunkrankheit ist.

12. Verwendung nach Anspruch 10, worin der Krankheitszustand aus Asthma, chronischer Bronchitis, chronischer Lungenentzündung, chronischer obstruktiver Atemwegerkrankung, atopischer Dermatitis, allergischer Rhinitis, Psoriasis, Arthritis, rheumatoider Arthritis, Gelenkentzündung, ulzeröser Kolitis, Morbus Crohn, atopischem Ekzem, Schlaganfall, Knochenresorptionserkrankung, Multipler Sklerose und entzündlicher Darmerkrankung ausgewählt ist.

13. Verwendung nach Anspruch 10, worin der Krankheitszustand aus Urtikaria, allergischer Konjunktivitis, Frühjahrskonjunktivitis, Augenentzündung, allergischen Reaktionen im Auge, eosinophilem Granulom, gichtartiger Arthritis und anderen arthritischen Zuständen, Atemnotsyndrom des Erwachsenen, Diabetes insipidus, Keratose, zerebraler Senilität, Multiinfarktdemenz, seniler Demenz, mit der Parkinson-Krankheit assoziierter Gedächtnisbeeinträchtigung, Depression, Herzstillstand, Claudicatio intermittens, rheumatischer Spondylitis, Osteoarthritis, Sepsis, septisch-toxischem Schock, Endotoxinschock, gramnegativer Sepsis, toxischem Schocksyndrom, akutem Atemnotsyndrom, Hirnmalaria, Silikose, pulmonärer Sarkoidose, Reperfusionsschädigung, Transplantat-Wirt-Reaktion, Allotransplantat-Abstoßung, Infektions-bedingtem Fieber oder Myalgie, Malaria, HIV, AIDS, ARC, Kachexie, Keloidbildung, Narbengewebebildung, Pyresis, systemischem Lupus erythematodes, Diabetes mellitus Typ 1, Bechet-Krankheit, Nephritis mit Purpura anaphylacta, chronischer Glomerulonephritis, Leukämie, Dyskinesia tarda, Hefe- oder Pilzinfektion, Zuständen, die einen Schutz des Magens erfordern, und neurogener Entzündungskrankheit, die mit Reizung und Schmerz assoziiert ist, ausgewählt ist.

14. Verwendung nach Anspruch 11, worin der Krankheitszustand Asthma ist.

15. Verwendung nach Anspruch 11, worin der Krankheitszustand chronisch obstruktive Atemwegerkrankung oder chronische Bronchitis ist.

## Revendications

1. Composé de la formule (i) : 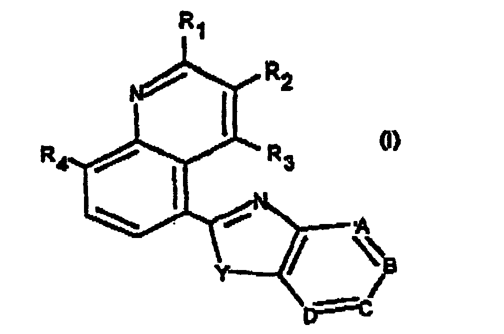 dans laquelle A, B, C et D sont identiques ou différents et sont chacun N, NO ou CR₅, à condition que pas plus de deux soient N ou NO ;
Y représente NR₇, O ou S ;
R₁, R₂ et R₃ sont identiques ou différents et chacun représente H, un alkyle ou CF₃ ;
R₄ représente un thioalkyle, un alcoxy ou cycloalcoxy en C_{1 à 6} facultativement substitué par un ou plusieurs halogènes ;
R₅ représente H, un halogène, un alkyle, CF₃, un hydroxy, OR₁₀, COR₁₁, SO₂R₁₁, SO₂NR₁₂R₁₃, NR₉R₁₀, CN, CO₂R₁₀, CONR₁₂R₁₃, NHSO₂CF₃ ou un tétrazolyle ;
R₇ représente H ou un alkyle en C_{1 à 6} ;
R₉ représente H, un alkyle, aryle, hétéroaryle, hétérocyclo, arylalkyle, hétéroarylalkyle, hétérocycloalkyle, alkylcarbonyle, alcoxycarbonyle, arylcarbonyle, hétéroarylcarbonyle, hétérocyclocarbonyle, alkylsulphonyle, arylsulphonyle, hétéroarylsulphonyle ou hétérocyclosulphonyle ;
R₁₀, R₁₂ et R₁₃ sont identiques ou différents et chacun représente H ou R₁₁, ou NR₁₂R₁₃ représente un noyau hétérocyclique facultativement substitué par R₁₄ ;
R₁₁ représente un alkyle, aryle, hétérocyclo, arylalkyle, hétéroarylalkyle ou hétérocycloalkyle ; et
R₁₄ représente un aryle, arylalkyle ou hétéroarylalkyle ;
ou un sel de ceux-ci acceptable sur le plan pharmaceutique.

2. Composé selon la revendication 1, dans lequel R₄ est un thioalkyle ou un alcoxy facultativement substitué ; et A, B, C et D sont chacun N ou CR₅, à condition que pas plus d'un soit N.

3. Composé selon la revendication 1 ou la revendication 2, dans lequel R₄ est un alcoxy facultativement substitué.

4. Composé selon la revendication 3, dans lequel un de A, B, C et D est CR₅ et les autres sont CH ;
R₁, R₂ et R₃ sont identiques ou différents et sont chacun H ou alkyle ;
R₅ et COOR₁₀, CONR₁₂, NHSO₂CF₃ ou tétrazolyle ; et
R₁₂ et R₁₃ sont chacun H ou R₁₁.

5. Composé selon la revendication 1, qui est l'acide 2-(8-méthoxyquinoléine-5-yl)-1H-benzimidazole-5-carboxylique sous la forme de son ester méthylique, de son dichlorhydrate, ou dichlorhydrate de l'ester méthylique.

6. Composé selon la revendication 1, qui est
2-(8-méthoxy-2-méthylquinoléine-5-yl)-5-méthoxy-1H-benzimidazole,
2-(8-méthoxy-2-trifluorométhylquinoléine-5-yl)-5-méthoxy-1H-benzimidazole,
2-(8-méthoxy-2-trifluorométhylquinoléine-5-yl)-5-méthoxy-1-méthyle-benzimidazole, ou
2-(8-méthoxy-2-trifluorométhyle-quinoléine-5-yl)-6-méthyle-benzimidazole.

7. Composé selon la revendication 1, qui est
2-(8-méthoxy-2-méthylquinoléine-5-yl)5-aza-1H-benzimidazole,
ester méthylique de l'acide 2-(8-méthoxy-2-méthylquinoléine-5-yl)-1H-benzimidazole-5-carboxylique,
2-(8-méthoxy-2-méthylquinoléine-5-yl)-5-méthoxybenzoxazole,
acide 2-(8-méthoxy-2-trifluorométhylquinoléine-5-yl)-1H-benzimidazole-5-carboxylique,
acide 2-(8-méthoxy-2-méthylquinoléine-5-yl)-1H-benzimidazole-5-carboxylique, ou
2-(8-méthoxy-2-trifluorométhylquinoléine-5-yl)-1H-benzimidazole-5-carboxamide.

8. Composé selon l'une quelconque des revendications précédentes, sous la forme d'un énantiomère de ceux-ci.

9. Composition pharmaceutique destinée à une utilisation thérapeutique comprenant un composé selon l'une quelconque des revendications précédentes et un vecteur ou un excipient acceptable sur le plan pharmaceutique.

10. Utilisation d'un composé selon l'une quelconque des revendications 1 à 8, pour la fabrication d'un médicament destiné à être utilisé dans le traitement d'un état pathologique qui peut être modulé par inhibition de la phosphodiestérase IV ou du facteur nécrosant des tumeurs, ou qui est un état pathologique associé à une fonction de la phosphodiestérase IV, à l'accumulation des éosinophiles ou à une fonction des éosinophiles.

11. Utilisation selon la revendication 10, dans laquelle l'état pathologique est une maladie inflammatoire ou une maladie autoimmune.

12. Utilisation selon la revendication 10, dans laquelle l'état pathologique est choisi parmi l'asthme, la bronchite chronique, une maladie pulmonaire inflammatoire chronique, la bronchopneumopathie chronique obstructive, la dermite atopique, la rhinite allergique, le psoriasis, l'arthrite, la polyarthrite rhumatoide, une inflammation des articulations, la recto-colite hémorragique, la maladie de Crohn, l'eczéma atopique, l'accident vasculaire cérébral, une maladie de résorption osseuse, la sclérose en plaque, et une affection abdominale inflammatoire.

13. Utilisation selon la revendication 10, dans laquelle l'état pathologique est choisi parmi l'urticaire, la conjonctive allergique, la conjonctivite printanière, une inflammation de l'oeil, les réactions allergiques oculaires, le granulome éosinophile, l'arthrite goutteuse et d'autres pathologies arthritiques, le syndrome de détresse respiratoire de l'adulte, le diabète insipide, la kératose, la sénilité cérébrale, la démence vasculaire, la démence sénile, les troubles de la mémoire liés à la maladie de Parkinson, la dépression, l'arrêt cardiaque, la claudication intermittente, la spondylite rhumatoïde, l'ostéoarthrite, la sepsie, le choc septique, le choc endotoxique, la sepsie à gram négatif, le syndrome du choc toxique, le syndrome de détresse respiratoire aiguë, malaria cérébrale, la silicose, la sarcoïdose pulmonaire, les lésions de reperfusion, une réaction hôte contre greffon, un rejet d'allogreffe, la fièvre ou la myalgie associées à une infection, la malaria, le VIH, le SIDA; le para-SIDA (ARC), la cachexie, la formation de chéloïdes, la formation de tissus cicatriciels, le pyrosis, le lupus érythémateux systémique, le diabète mellitus de type I, la maladie de Bechet, le purpura anaphylactoïde, la néphrite, la glomérulonéphrite chronique, la leucémie, la dyskinésie tardive, les levuroses ou les infections fongiques, les pathologies nécessitant une gastroprotection, une pathologie inflammatoire neurogène associée à des irritations et à des douleurs.

14. Utilisation selon la revendication 11, dans laquelle l'état pathologique est l'asthme.

15. Utilisation selon la revendication 11, dans laquelle l'état pathologique est la broncho-pneumopathie chronique obstructive ou la bronchite chronique.
